# EUROPEAN PATENT SPECIFICATION

(11) **EP 3 176 148 B1**
(45) Date of publication and mention of the grant of the patent: **07.10.2020**
(21) Application number: 15827679.0
(22) Date of filing: 24.07.2015
(51) Int. Cl.: C07C 29/151, C01B 3/34, C07C 31/08

(54) **ALCOHOL PRODUCTION DEVICE AND ALCOHOL PRODUCTION METHOD**
ALKOHOLHERSTELLUNGSVORRICHTUNG UND ALKOHOLHERSTELLUNGSVERFAHREN
DISPOSITIF DE PRODUCTION D'ALCOOL ET PROCÉDÉ DE PRODUCTION D'ALCOOL

(30) Priority: 30.07.2014 JP 2014155501
(43) Date of publication of application: 07.06.2017
(73) Proprietor: Sekisui Chemical Co., Ltd., Osaka-shi, Osaka 530-8565 (JP)
(72) Inventor: KOMA, Satoshi, Tokyo 105-8450 (JP)
(74) Representative: Ter Meer Steinmeister & Partner
(86) International application number: PCT/JP2015/071140
(87) International publication number: WO 2016/017549

(56) References cited:
- EP-A2- 2 229 339
- EP-A2- 2 483 371
- WO-A1-2013/033812
- WO-A1-2013/186886
- JP-A- 2008 544 943
- JP-A- 2012 246 232
- US-A- 6 114 400
- US-A1- 2007 010 589

## Description

### TECHNICAL FIELD

The present invention relates to an apparatus and a method for producing an alcohol.

### DESCRIPTION OF RELATED ART

Conventionally, a method is known in which ethanol is produced by reacting a synthesis gas obtained from biomass in the presence of a catalyst (see Patent Document 1). The catalytic reaction of a synthesis gas may result in generation of by-product such as methane, ethane and ethylene, in addition to ethanol as a target product. Patent Document 1 describes the reformation of such by-products into carbon monoxide and hydrogen. US2007/0010589 discloses a carbonaceous feedstock to alcohol conversion process.

### DOCUMENTS OF RELATED ART

### [Patent Document]

[Patent Document 1] Japanese Unexamined Patent Application Publication No. 2012-149089

### SUMMARY OF THE INVENTION

### PROBLEMS TO BE SOLVED BY THE INVENTION

It has been desired to improve energy efficiency in production of an alcohol such as bioethanol.

It is a primary object of the present invention to provide an apparatus and a method which can be used for improving energy efficiency in production of an alcohol.

### MEANS TO SOLVE THE PROBLEMS

With respect to the apparatus of the present invention for producing an alcohol the apparatus is according with claim 1.

### DETAILED DESCRIPTION OF THE INVENTION

With respect to the method of the present invention for producing a chemical product, the method comprises the steps as from claim 2.

### EFFECT OF THE INVENTION

As described above, the present invention can provide an apparatus and a method which can be used for improving energy efficiency in production of an alcohol.

### BRIEF DESCRIPTION OF THE DRAWINGS

FIG. 1 is a schematic view of the apparatus for producing an alcohol according to a first embodiment of the present invention.
FIG. 2 is a schematic view of the apparatus for producing an alcohol according to a second embodiment of the present invention.

### DESCRIPTION OF THE EMBODIMENTS

Hereinbelow, the present invention will be described in detail with reference to preferred embodiments of the present invention. However, these embodiments are only examples. The present invention is in no way limited by these embodiments.

### (First Embodiment)

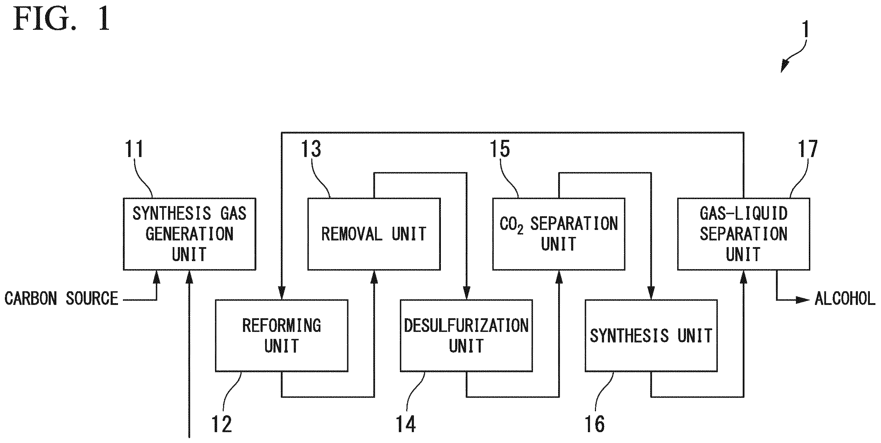

FIG. 1 is a schematic view of the apparatus for producing an alcohol according to a first embodiment of the present invention. The apparatus 1 shown in FIG. 1 is an apparatus for producing an alcohol from organic waste, biomass and the like.

The apparatus 1 comprises a synthesis gas generation unit 11. A non-fossil organic resource (i.e., carbon source) such as organic wastes, biomass or the like is fed into the synthesis gas generation unit 11. The synthesis gas generation unit 11 generates a synthesis gas by partial oxidation of these carbon sources. Generally, the partial oxidation of a carbon source results in generation of by-products such as hydrocarbons, and carbon dioxide. Therefore, the synthesis gas obtained in the synthesis gas generation unit 11 contains hydrocarbons, carbon dioxide, and the like, in addition to carbon monoxide and hydrogen. Subsequently, the synthesis gas generation unit 11 feeds the obtained synthesis gas into a reforming unit 12. The hydrocarbons contained in the synthesis gas as by-products in the synthesis gas generation unit 11 include hydrocarbons having 1 to 6 carbon atoms. Specifically, the hydrocarbons include a large amount of methane.

The reforming unit 12 reforms the hydrocarbons contained in the synthesis gas into carbon monoxide and hydrogen. Subsequently, the reforming unit 12 feeds the reformed synthesis gas into a removal unit 13.

The removal unit 13 removes impurities contained in the synthesis gas. Specifically, in the removal unit 13, the impurities contained in the synthesis gas are dissolved in a solution or the like so as to remove the impurities from the synthesis gas. Subsequently, the resulting synthesis gas free of impurities is fed from the removal unit 13 into a desulfurization unit 14.

The desulfurization unit 14 separates sulfur content off from the synthesis gas so as to obtain a desulfurized synthesis gas. Subsequently, the desulfurization unit 14 feeds the desulfurized synthesis gas into a carbon dioxide separation unit 15.

The carbon dioxide separation unit 15 separates carbon dioxide off from the synthesis gas so as to obtain a carbon dioxide-free synthesis gas. Subsequently, the carbon dioxide separation unit 15 feeds the carbon dioxide-free synthesis gas into a synthesis unit 16.

The synthesis unit 16 subjects the synthesis gas to catalytic reaction so as to synthesize an alcohol and the like by catalytic reaction of carbon monoxide and hydrogen, which are contained in the resultant synthesis gas. As an example of a catalyst used in the catalytic reaction, a four-way catalyst composed of rhodium, manganese, lithium and magnesium, and the like, can be mentioned. Generally, the catalytic reaction of the synthesis gas results in generation of by-products such as hydrocarbons. Accordingly, the synthesis unit 16 feeds the resultant components including the target alcohol as well as by-products into the gas-liquid separation unit 17.

The gas-liquid separation unit 17 separates the components into gas and liquid. Consequently, in the gas-liquid separation unit 17, the liquid components including an alcohol are obtained. The gas-liquid separation unit 17 may be connected to a purification unit for purifying an alcohol from the liquid components.

In the gas-liquid separation unit 17, the separated gas includes unreacted carbon monoxide and hydrogen, and hydrocarbons and the like. The gas-liquid separation unit 17 feeds these gas components to the reforming unit 12.

The reforming unit 12 reforms hydrocarbons contained in the separated gas into carbon monoxide and hydrogen. Therefore, the carbon monoxide and hydrogen, which are obtained by the reforming of the hydrocarbons contained in the separated gas, and the unreacted carbon monoxide and hydrogen contained also in the separated gas, are fed to the synthesizing unit 16 and used for synthesizing a chemical product such as an alcohol.

The reforming reaction performed in the reforming unit 12 is an endothermic reaction. Accordingly, it is necessary to supply heat to the reforming unit 12. In the apparatus 1, the heat exhausted from the synthesis gas generation unit 11 is supplied to the reforming unit 12. The reforming unit 12 reforms the hydrocarbons by utilizing heat exhausted from the synthesis gas generation unit 11. Consequently, the amount of external heat to be supplied to the reforming unit 12 can be reduced. Thus, the first embodiment of the present invention can provide an apparatus and a method which can be used for improving energy efficiency in production of an alcohol.

Hereinbelow, the present invention will be described in detail with reference to further preferred embodiments of the present invention. In the following descriptions, components having substantially the same functions as those in the first embodiment are denoted with the same reference numerals, and the descriptions thereof are omitted.

### (Second Embodiment)

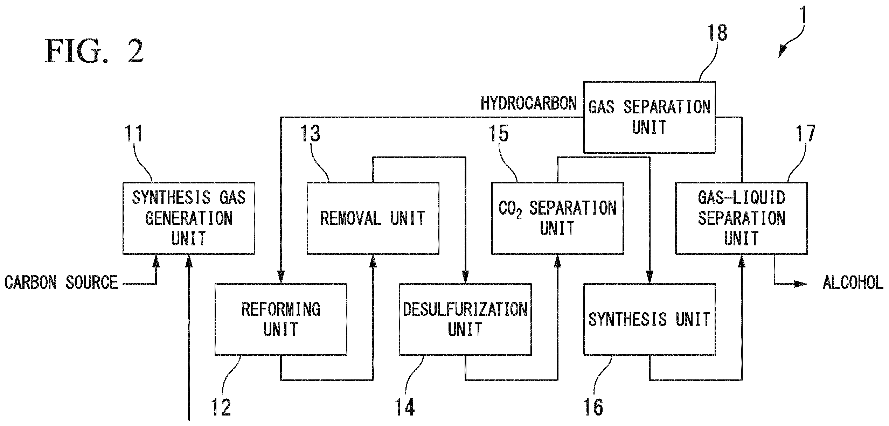

FIG. 2 is a schematic view of the apparatus for producing a chemical product according to a second embodiment of the present invention. In the second embodiment, a gas separation unit 18 is connected to the gas-liquid separation unit 17. The gas separated in the gas-liquid separation unit 17 is fed into the gas separation unit 18. The gas separation unit 18 separates the gas into hydrocarbons and other components. The gas separation unit 18 feeds the obtained hydrocarbons into the reforming unit 12. The gas separation unit 18 feeds the obtained other components including unreacted carbon monoxide and hydrogen into the synthesis unit 16. Thus, this embodiment of the present invention makes it possible to feed only the hydrocarbons separately from other gas components separated by the gas-liquid separation unit 17 into the reforming unit 12. Accordingly, the reforming of the hydrocarbons can be performed more efficiently.

### DESCRIPTION OF THE REFERENCE SIGNS

- 1:: Apparatus
- 11:: Synthesis gas generation unit
- 12:: Reforming unit
- 13:: Removal unit
- 14:: Desulfurization unit
- 15:: Carbon dioxide separation unit
- 16:: Synthesis unit
- 17:: Gas-liquid separation unit
- 18:: Gas separation unit

## Claims

1. An apparatus (1) for producing an alcohol from organic waste and/or biomass, the apparatus (1) comprising:
a synthesis gas generation unit (11) which is configured to generate a synthesis gas by partial oxidation of a carbon source, wherein the carbon source is selected from the group consisting of organic waste, biomass and combinations thereof, and wherein the generated synthesis gas contains carbon monoxide, hydrogen and by-products, the by-products being selected from the group consisting of hydrocarbons having 1 to 6 carbon atoms and including a large amount of methane, carbon dioxide and combinations thereof;
a reforming unit (12) which is configured to receive the synthesis gas that has been obtained in the synthesis gas generation unit (11) as a feed and which is further configured to reform hydrocarbons into carbon monoxide and hydrogen;
a removal unit (13) which is configured to receive the reformed synthesis gas that has been obtained in the reforming unit (12) as a feed and which is further configured to remove impurities contained in the reformed synthesis gas by dissolving the impurities in a solution;
a desulfurization unit (14) which is configured to receive the synthesis gas free of impurities that has been obtained in the removal unit (13) as a feed and which is further configured to separate sulfur content from the synthesis gas so as to obtain a desulfurized synthesis gas;
a carbon dioxide separation unit (15) which is configured to receive the desulfurized synthesis gas that has been obtained in the desulfurization unit (14) as a feed and which is further configured to separate carbon dioxide off from the synthesis gas so as to obtain a carbon dioxide-free synthesis gas;
a synthesis unit (16) which is configured to receive the carbon dioxide-free synthesis gas that has been obtained in the carbon dioxide separation unit (15) as a feed and which is further configured to subject the carbon monoxide and the hydrogen contained in the synthesis gas to a catalytic reaction in order to synthesize resultant components, wherein the resultant components include an alcohol and hydrocarbons as by-products; and
a gas-liquid separation unit (17) which is configured to receive the resultant components that have been obtained in the synthesis unit (16) as a feed and which is further configured to separate the resultant components into a gas and a liquid, wherein the liquid includes the alcohol and wherein the gas includes unreacted carbon monoxide, hydrogen and hydrocarbons;
wherein the reforming unit (12) is further configured to
receive the separated gas obtained in the gas-liquid separation unit (17) as a feed, and to reform the hydrocarbons by utilizing heat exhausted from the synthesis gas generation unit (11).

2. A method for producing an alcohol from organic waste and/or biomass, wherein the method comprises the following sequence of steps:
a) a step of performing partial oxidation of a carbon source to generate a synthesis gas, wherein the carbon source is selected from the group consisting of organic waste, biomass and combinations thereof, and wherein the generated synthesis gas contains carbon monoxide, hydrogen and by-products, the by-products being selected from the group consisting of hydrocarbons having 1 to 6 carbon atoms and including a large amount of methane, carbon dioxide and combinations thereof;
b) a step of reforming the hydrocarbons into carbon monoxide and hydrogen in order to obtain a reformed synthesis gas;
c) a step of removing impurities contained in the reformed synthesis gas by dissolving the impurities in a solution in order to obtain a synthesis gas free of impurities;
d) a step of separating sulfur content from the synthesis gas free of impurities so as to obtain a desulfurized synthesis gas;
e) a step of separating carbon dioxide off from the desulfurized synthesis gas so as to obtain a carbon dioxide-free synthesis gas;
f) a step of subjecting the carbon monoxide and the hydrogen contained in the synthesis gas to a catalytic reaction in order to synthesize resultant components, wherein the resultant components include an alcohol, and hydrocarbons as by-products;
g) a step of separating the resultant components into a gas and a liquid, wherein the liquid includes the alcohol and wherein the gas includes unreacted carbon monoxide, hydrogen and hydrocarbons;
h) a step of subjecting the gas to step b),
wherein step b) is performed by utilizing heat exhausted from the step a).

## Patentansprüche

1. Vorrichtung (1) zum Erzeugen eines Alkohols aus organischem Abfall und/oder aus Biomasse, wobei die Vorrichtung (1) Folgendes umfasst:
eine Synthesegaserzeugungseinheit (11), die konfiguriert ist, durch teilweise Oxidation einer Kohlenstoffquelle ein Synthesegas zu erzeugen, wobei die Kohlenstoffquelle aus der Gruppe gewählt ist, die aus organischem Abfall, Biomasse und Kombinationen davon besteht, und wobei das erzeugte Synthesegas Kohlenmonoxid, Wasserstoff und Nebenprodukte enthält, wobei die Nebenprodukte aus der Gruppe gewählt sind, die aus Kohlenwasserstoffen mit 1 bis 6 Kohlenstoffatomen besteht, und eine große Menge Methan, Kohlendioxid und Kombinationen davon enthalten;
eine Umwandlungseinheit (12), die konfiguriert ist, das Synthesegas, das in der Synthesegaserzeugungseinheit (11) erhalten worden ist, als eine Beschickung zu empfangen, und die ferner konfiguriert ist, Kohlenwasserstoffe in Kohlenmonoxid und Wasserstoff umzuwandeln;
eine Entfernungseinheit (13), die konfiguriert ist, das umgewandelte Synthesegas, das in der Umwandlungseinheit (12) erhalten worden ist, als eine Beschickung zu empfangen, und die ferner konfiguriert ist, Verunreinigungen, die in dem umgewandelten Synthesegas enthalten sind, zu entfernen, indem die Verunreinigungen in einer Lösung gelöst werden;
eine Entschwefelungseinheit (14), die konfiguriert ist, das verunreinigungsfreie Synthesegas, das in der Entfernungseinheit (13) erhalten worden ist, als eine Beschickung zu empfangen, und die ferner konfiguriert ist, einen Schwefelgehalt vom Synthesegas zu trennen, um ein entschwefeltes Synthesegas zu erhalten;
eine Kohlendioxidtrenneinheit (15), die konfiguriert ist, das entschwefelte Synthesegas, das in der Entschwefelungseinheit (14) erhalten worden ist, als eine Beschickung zu empfangen, und die ferner konfiguriert ist, Kohlendioxid vom Synthesegas abzusondern, um ein kohlendioxidfreies Synthesegas zu erhalten;
eine Syntheseeinheit (16), die konfiguriert ist, das kohlendioxidfreie Synthesegas, das in der Kohlendioxidtrenneinheit (15) erhalten worden ist, als eine Beschickung zu empfangen, und die ferner konfiguriert ist, das Kohlenmonoxid und den Wasserstoff, die im Synthesegas enthalten sind, mit einer katalytischen Reaktion zu beaufschlagen, um resultierende Komponenten zu synthetisieren, wobei die resultierenden Komponenten einen Alkohol und Kohlenwasserstoffe als Nebenprodukte enthalten; und
eine Gas/Flüssigkeits-Trenneinheit (17), die konfiguriert ist, die resultierenden Komponenten, die in der Syntheseeinheit (16) erhalten worden sind, als eine Beschickung zu empfangen. und die ferner konfiguriert ist, die resultierenden Komponenten in ein Gas und eine Flüssigkeit zu trennen, wobei die Flüssigkeit den Alkohol enthält und wobei das Gas Kohlenmonoxid, Wasserstoff und Kohlenwasserstoffe, die nicht reagiert haben, enthält;
wobei die Umwandlungseinheit (12) ferner konfiguriert ist zum
Empfangen des getrennten Gases, das in der Gas/Flüssigkeits-Trenneinheit (17) erhalten wird, als eine Beschickung und Umwandeln der Kohlenwasserstoffe durch Nutzen von Wärme, die von der Synthesegaserzeugungseinheit (11) abgegeben wird.

2. Verfahren zum Erzeugen eines Alkohols aus organischem Abfall und/oder Biomasse, wobei das Verfahren die folgende Abfolge von Schritten umfasst:
a) einen Schritt des Durchführens einer teilweisen Oxidation einer Kohlenstoffquelle, um ein Synthesegas zu erzeugen, wobei die Kohlenstoffquelle aus der Gruppe gewählt wird, die aus organischem Abfall, Biomasse und Kombinationen davon besteht, und wobei das erzeugte Synthesegas Kohlenmonoxid, Wasserstoff und Nebenprodukte enthält, wobei die Nebenprodukte aus der Gruppe gewählt sind, die aus Kohlenwasserstoffen mit 1 bis 6 Kohlenstoffatomen besteht, und eine große Menge Methan, Kohlendioxid und Kombinationen davon enthalten;
b) einen Schritt des Umwandelns der Kohlenwasserstoffe in Kohlenmonoxid und Wasserstoff, um ein umgewandeltes Synthesegas zu erhalten;
c) einen Schritt des Entfernens von Verunreinigungen, die in dem umgewandelten Synthesegas enthalten sind, durch Lösen der Verunreinigungen in einer Lösung, um ein verunreinigungsfreies Synthesegas zu erhalten;
d) einen Schritt des Trennens eines Schwefelgehalts von dem verunreinigungsfreien Synthesegas, um ein entschwefeltes Synthesegas zu erhalten;
e) einen Schritt des Absonderns von Kohlendioxid von dem entschwefelten Synthesegas, um ein kohlendioxidfreies Synthesegas zu erhalten;
f) einen Schritt des Beaufschlagens des Kohlenmonoxids und des Wasserstoffs, die im Synthesegas enthalten sind, mit einer katalytischen Reaktion, um resultierende Komponenten zu synthetisieren, wobei die resultierenden Komponenten einen Alkohol und Kohlenwasserstoffe als Nebenprodukte enthalten;
g) einen Schritt des Trennens der resultierenden Komponenten in ein Gas und eine Flüssigkeit, wobei die Flüssigkeit den Alkohol enthält und wobei das Gas Kohlenmonoxid, Wasserstoff und Kohlenwasserstoffe, die nicht reagiert haben, enthält;
h) einen Schritt, das Gas mit dem Schritt b) zu beaufschlagen,
wobei der Schritt b) durch Nutzen von Wärme, die aus dem Schritt a) abgegeben wird, durchgeführt wird.

## Revendications

1. Appareil (1) pour produire un alcool à partir de déchets organiques et/ou d'une biomasse, l'appareil (1) comportant :
une unité de génération de gaz de synthèse (11) qui est configurée pour générer un gaz de synthèse par oxydation partielle d'une source de carbone, dans lequel la source de carbone est choisie parmi le groupe constitué de déchets organiques, d'une biomasse et de combinaison de ceux-ci, et dans lequel le gaz de synthèse généré contient du monoxyde de carbone, de l'hydrogène et des sous-produits, les sous-produits étant choisis parmi le groupe constitué d'hydrocarbures possédant 1 à 6 atomes de carbone et incluant une grande quantité de méthane, de dioxyde de carbone et de combinaisons de ceux-ci ;
une unité de reformage (12) qui est configurée pour recevoir le gaz de synthèse qui a été obtenu dans l'unité de génération de gaz de synthèse (11) comme une charge d'alimentation et qui est en outre configurée pour reformer des hydrocarbures en monoxyde de carbone et en hydrogène ;
une unité d'extraction (13) qui est configurée pour recevoir le gaz de synthèse reformé qui a été obtenu dans l'unité de reformage (12) comme une charge d'alimentation et qui est en outre configurée pour éliminer des impuretés contenues dans le gaz de synthèse reformé en dissolvant les impuretés dans une solution ;
une unité de désulfuration (14) qui est configurée pour recevoir le gaz de synthèse exempt d'impuretés qui a été obtenu dans l'unité d'extraction (13) comme une charge d'alimentation et qui est en outre configurée pour séparer la quantité de soufre du gaz de synthèse de manière à obtenir un gaz de synthèse désulfuré ;
une unité de séparation du dioxyde de carbone (15) qui est configurée pour recevoir le gaz de synthèse désulfuré qui a été obtenu dans l'unité de désulfuration (14) comme une charge d'alimentation et qui est en outre configurée pour séparer le dioxyde de carbone du gaz de synthèse de manière à obtenir un gaz de synthèse exempt de dioxyde de carbone ;
une unité de synthèse (16) qui est configurée pour recevoir le gaz de synthèse exempt de dioxyde de carbone qui a été obtenu dans l'unité de séparation du dioxyde de carbone (15) comme une charge d'alimentation et qui est en outre configurée pour soumettre le monoxyde de carbone et l'hydrogène contenus dans le gaz de synthèse à une réaction catalytique afin de synthétiser des composants résultants, dans lequel les composants résultants incluent un alcool et des hydrocarbures en tant que sous-produits ; et
une unité de séparation de gaz-liquide (17) qui est configurée pour recevoir les composants résultants qui ont été obtenus dans l'unité de synthèse (16) comme une charge d'alimentation et qui est en outre configurée pour séparer les composants résultants en un gaz et un liquide, dans lequel le liquide inclut l'alcool et dans lequel le gaz inclut du monoxyde de carbone, de l'hydrogène et des hydrocarbures n'ayant pas réagi ;
dans lequel l'unité de reformage (12) est en outre configurée pour recevoir le gaz séparé obtenu dans l'unité de séparation de gaz-liquide (17) comme une charge d'alimentation, et pour reformer les hydrocarbures en utilisant de la chaleur évacuée à partir de l'unité de génération de gaz de synthèse (11).

2. Procédé pour produire un alcool à partir de déchets organiques et/ou d'une biomasse, dans lequel le procédé comporte la séquence d'étapes suivante :
a) une étape consistant à réaliser une oxydation partielle d'une source de carbone pour générer un gaz de synthèse, dans lequel la source de carbone est choisie parmi le groupe constitué de déchets organiques, d'une biomasse et de combinaisons de ceux-ci, et dans lequel le gaz de synthèse généré contient du monoxyde de carbone, de l'hydrogène et des sous-produits, les sous-produits étant choisis parmi le groupe constitué d'hydrocarbures possédant 1 à 6 atomes de carbone et incluant une grande quantité de méthane, de dioxyde de carbone et de combinaisons de ceux-ci ;
b) une étape consistant à reformer les hydrocarbures en monoxyde de carbone et en hydrogène afin d'obtenir un gaz de synthèse reformé ;
c) une étape consistant à éliminer des impuretés contenues dans le gaz de synthèse reformé en dissolvant les impuretés dans une solution afin d'obtenir un gaz de synthèse exempt d'impuretés ;
d) une étape consistant à séparer la quantité de soufre du gaz de synthèse exempt d'impuretés de manière à obtenir un gaz de synthèse désulfuré ;
e) une étape consistant à séparer le dioxyde de carbone du gaz de synthèse désulfuré de manière à obtenir un gaz de synthèse exempt de dioxyde de carbone ;
f) une étape consistant à soumettre le monoxyde de carbone et l'hydrogène contenu dans le gaz de synthèse à une réaction catalytique afin de synthétiser des composants résultants, dans lequel les composants résultants incluent un alcool et des hydrocarbures en tant que sous-produits ;
g) une étape consistant à séparer les composants résultants en un gaz et un liquide, dans lequel le liquide inclut l'alcool et dans lequel le gaz inclut du monoxyde de carbone, de l'hydrogène et des hydrocarbures n'ayant pas réagi ;
h) une étape consistant à soumettre le gaz à l'étape b),
dans lequel l'étape b) est réalisée en utilisant de la chaleur évacuée à partir de l'étape a).
